(19) Europäisches Patentamt
European Patent Office
Office européen des brevets

(11) Veröffentlichungsnummer : **0 096 657**
**B1**

(12) # EUROPÄISCHE PATENTSCHRIFT

(45) Veröffentlichungstag der Patentschrift : **05.04.89**

(51) Int. Cl.[4] : **C 07 D239/30**, C 07 D239/42, C 07 D405/04, C 07 D409/04, C 07 D403/04, C 07 D401/04, C 07 D403/10, C 07 D417/04, A 01 N 25/32

(21) Anmeldenummer : **83810229.1**

(22) Anmeldetag : **02.06.83**

(54) **2-Phenyl-, 2-Naphthyl- und 2-Heterocyclylpyrimidine als Gegenmittel zum Schützen von Kulturpflanzen vor durch Herbizide verursachte phytotoxische Schäden.**

(30) Priorität : **08.06.82 CH 3526/82**
**21.06.82 CH 3795/82**
**16.11.82 CH 6665/82**

(43) Veröffentlichungstag der Anmeldung : **21.12.83 Patentblatt 83/51**

(45) Bekanntmachung des Hinweises auf die Patenterteilung : **05.04.89 Patentblatt 89/14**

(84) Benannte Vertragsstaaten : **BE CH DE FR GB IT LI NL**

(56) Entgegenhaltungen :
**EP-A- 0 055 693**
**JOURNAL OF THE CHEMICAL SOCIETY, 1952, Seiten 328-333, Absatz 68; J.A. HENDRY et al.: "New cytotoxic agents with tumour-inhibitory activity. Part I. Some aziridinopyrimidine derivatives"**
**COMPTES RENDUS ACAD. AC., Band 281, Nr. 1, 7. Juli 1975, Serie C, Seiten 39-41, Paris, FR; JAN PANKIEWICZ et al.: "Propriétés de nitriles substitués ou non en ortho par un groupe hydroxy en série thiophénique et furannique"**
**JOURNAL OF ORGANIC CHEMISTRY, Band 32, Nr. 5, Mai 1967, Seiten 1591-1595, Washington, US; JOHN J. LAFFERTY et al.: "The preparation and properties of certain pyridylpyrimidines and bidiazines as potential chelating agents for iron(II)"**
**Austr. J. Chem. 34 (1981), 1353**

(73) Patentinhaber : **CIBA-GEIGY AG**
**Klybeckstrasse 141**
**CH-4002 Basel (CH)**

(72) Erfinder : **Brunner, Hans-Georg, Dr.**
**Wannenstrasse 14**
**CH-4415 Lausen (CH)**
Erfinder : **Burdeska, Kurt, Dr.**
**Paracelsusstrasse 64**
**CH-4058 Basel (CH)**
Erfinder : **Föry, Werner, Dr.**
**Benkenstrasse 65**
**CH-4054 Basel (CH)**

**EP 0 096 657 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 2-Phenyl-, 2-Naphthyl- und 2-Heterocyclylpyrimidine, welche sich als Gegenmittel zum Schützen von Kulturpflanzen vor durch Herbizide verursachte phytotoxische Schäden eignen. Debei werden 2-Phenyl-, 2-Naphthyl- und 2-Heterocyclylpyrimidine gleichzeitig oder in kurzer Folge mit dem Herbizid den Kulturpflanzungen appliziert. Man kann auch ein Mittel anwenden, welches sowohl das Herbizid wie das 2-Phenyl-, 2-Naphthyl- oder 2-Heterocyclylpyrimidin enthält oder man kann die Samen oder das Saatgut der Kulturpflanze mit dem 2-Phenyl-, 2-Naphthyl- oder 2-Heteropcyclylpyrimidin vorbehandeln (beizen) und nachher die gesäte oder aufgelaufene Kultur mit dem Herbizid applizieren. Die Erfindung betrifft auch Mittel, welche die 2-Phenyl-, 2-Naphthyl- oder 2-Heterocyclylpyrimidine enthalten sowie ihre Verwendung.

Es ist bekannt, dass Herbizide aus den verschiedensten Stoffklassen, wie Triazine, Harnstoffderivate, Carbamate, Thiolcarbamate, Halogenacetanilide, Halogenphenoxyessigsäure usw. bei der Anwendung in wirksamer Dosis gelegentlich neben den zu bekämpfenden Unkräutern auch die Kulturpflanzen in gewissem Masse schädigen. Ueberdosen werden oft ungewollt und zufälligerweise appliziert, wenn sich Randzonen beim streifenweisen Spritzen überdecken, sei es durch Windeinwirkung oder durch falsches Einschätzen der Breitenwirkung des Spritzgerätes. Es können klimatische Verhältnisse oder eine Bodenbeschaffenheit vorliegen, so dass die für normale Bedingungen empfohlene Herbizidmenge als Ueberdosis wirkt. Die Qualität des Saatgutes kann bei der Herbizidverträglichkeit auch eine Rolle spielen. Um diesem Problem zu begegnen, sind schon verschiedene Stoffe vorgeschlagen worden, welche befähigt sind, die schädigende Wirkung des Herbizids auf die Kulturpflanze spezifisch zu antagonisieren, d. h. die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, d. h. ein bestimmtes Gegenmittel eignet sich oft nur für eine bestimmte Kulturpflanze und einige wenige herbizide Stoffklassen.

Die 2-Phenyl-, 2-Naphthyl und 2-Heterocyclyl-pyrimidine entsprechen der Formel (I)

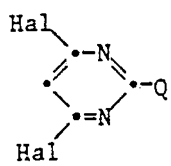

(I)

worin Hal ein Halogenatom, Q einen Phenylrest

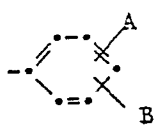

einen unsubstituierten α-Naphthylrest oder einen ungesättigten oder teilweise gesättigten oder auch benzannellierten 5-6 gliedrigen Heterocyclus bedeutet.

Der unsubstituiert oder ein- oder mehrfach durch Halogen, $C_1$-$C_6$-Alkyl, $C_1$-$C_6$-Alkoxy oder Dimethylamino substituiert ist und worin im Phenylrest Q A einen Rest Phenyl, Phenoxy, Phenyl-$C_{2-6}$-alkinyl, Benzylidenimino, Benzoylamido, Pyrrol-1-yl oder einen $C_2$-$C_6$-Alkenylrest, der durch Cyan und/oder Carboxy substituiert ist oder einen $C_1$-$C_4$-Hydroxyalkylaminorest, und

B Wasserstoff oder

A und B zusammen die Methylendioxygruppe —O—$CH_2$—O bedeuten, mit der Massgabe, dass wenn Hal Chlor bedeutet, Q nicht ein unsubstituierten Pyridyl-, Furyl- oder Thienylrest oder ein 2-Dimethylamino-4-pyrimidinylrest sein kann.

Einige Dichlorpyrimidinderivate sind ohne Verwendungsangabe bereits bekannt. 2-β-Naphthyl-4,6-dichloropyrimidin ist z. B. im J. Chem. Soc. 1952 328-333, 2-Furyl- und 2-Thienyl-4,6-dichlorpyrimidin in den Comptes Rendus 281 (1975) C 39-41, 2-Pyridyl-4,6-diphenyl-4,6-dichloropyrimidin im J. Org. Chem. 32 (1967) 1591-1595 und das Bipyrimidin 2-(2'-Dimethylamino-pyrimidin-4'-yl)-4,6-dichloropyrimidin im Austr. J. Chem. 34 (1981) 1353-1359 beschrieben worden. EP-A-55 693 ist eine frühere Anmeldung und betrifft bestimmte Phenylpyrimidine.

Der Ausdruck Alkyl allein oder als Teil eines Substituenten umfasst verzweigte oder unverzweigte Alkylgruppen, welche die angegebene Anzahl Kohlenstoffatome enthalten. Beispiele sind Methyl, Aethyl, Propyl, Isopropyl, Butyl, Isobutyl, sec.-Butyl, tert.-Butyl, sowie die höheren Homologen Amyl, Isoamyl, Hexyl, samt ihren Isomeren. Entsprechend können die Alkenyl- und Alkinylgruppen geradkettig oder verzweigt sein. Die Alkenyl- und Alkinylreste können verzweigt oder unverzweigt sein und eine oder mehrere zwei- oder dreifach-Bindungen aufweisen.

Ungesättigte oder teilweise gesättigte und/oder benzannelierte 5-6 gliedrige Heterocyclen entsprechend der Definition von Q sind beispielsweise Furan, Pyran, Thiophen, Thiazol, Pyridin, Pyrrolin, Oxazol, Isoxazol, Thioxazol, Isothiazol, Thiadiazol, Oxthiazol, Pyrrol, Imidazol, Pyrazol, Pyrazin, Pyrimidin, Pyridazin, 1,3,5-Triazin, 1,2,4-Triazin, 1,2,4-Triazol, 1,2,3-Triazol, 1,3,4-Triazol, Oxdiazol, Oxazin, Furazan, Pyridin-N-oxid, Thiophen-5-oxid, Benzthiophen, Benzofuran, Isobenzofuran, Chromen, Chroman, Indol, Isoindol, Indazol, Chinolin, Isochinolin, Phthalazin, Chinoxalin, Chinazolin, Cinnolin, Benzthiazol, Benzimidazol.

Diese Ringe sind mit dem Pyrimidinring über ein Kohlenstoffatom gebunden oder im Falle von N-Heterocyclen gegebenenfalls auch über ein Stickstoffatom. Sie können unsubstituiert oder wie oben angegeben substituiert sein.

Die Phenylpyrimidine der Formel (I) eignen sich hervorragend, Kulturpflanzen wie Kulturhirse, Reis, Mais, Getreidearten (Weizen, Roggen, Gerste, Hafer), Baumwolle, Zuckerrüben, Zuckerrohr, Soja vor dem Angriff von Pflanzenaggressiven Agrarchemikalien, insbesondere von Herbiziden verschiedenster Stoffklassen, wie Triazinone, Chloracetaniliden, Pyridyloxyphenoxypropionsäureestern, zu schützen, sofern diese nicht oder nicht genügend selektiv wirken, also neben den zu bekämpfenden Unkräutern auch die Kulturpflanzen mehr oder weniger schädigen. Die Erfindung betrifft auch Mittel, welche diese Phenylpyrimidine der Formel (I) zusammen mit Herbiziden enthalten.

Unter den Verbindungen der Formel (I) haben sich diejenigen bewährt, welche folgenden Untergruppen entsprechen :

Phenylpyrimidine der Formel (Ia)

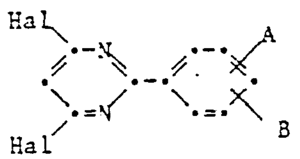

(Ia)

worin Hal Chlor oder Brom A einen Rest Phenyl, Phenyloxy, Phenyl-$C_{2-6}$-alkinyl, Benzylidenimino, ein durch Cyan und/oder COOH, substituiertes $C_2$-$C_6$ Alkenyl oder $C_{1-4}$ Hydroxyalkylaminorest B Wasserstoff und A und B zusammen die Methylendioxygruppe $—O—CH_2—O—$ bedeuten.

Ebenfalls guter Schutz wird mit den Verbindungen der Formel (I) erzielt, in denen Q einen 5-6-gliedrigen, ungesättigten oder teilweise gesättigten und/oder benzannelierten Heterocyclus bildet, der unsubstituiert oder wie oben angegeben substituiert ist.

Die beste Schutzwirkung zeigten diejenigen Verbindungen der Formel (I), in denen Q einen unsubstituierten α-Naphthyl-, oder substituierten Furyl-, Thienyl- oder Pyridylrest verkörpert ist, insbesondere auch die Verbindungen :

4,6-Dichlor-2-(1'-naphthyl)-pyrimidin,
4,6-Dichlor-2-(5'-brompyrid-3'-yl)-pyrimidin
4,6-Dichlor-2-(4',6'-dimethylpyrimidin-2'-yl)-pyrimidin
4,6-Dichlor-2-(3'-chinolyl)-pyrimidin
4,6-Dichlor-2-(2'-methyl-thien-5'-yl)-pyrimidin
4,5-Dichlor-2-(2'-pyrrolyl)-pyrimidin
4,6-Dichlor-2-(1'-methyl-pyrrol-2'-yl)-pyrimidin
2-(3,4-Methylendioxyphenyl)-4,6-dichlorpyrimidin,
2-(4-Benzylideniminophenyl)-4,6-dichlorpyrimidin,
2-(4-Dihydroxyäthylaminophenyl)-4,6-dichlorpyrimidin,
2-[4-(2-Cyano-2'-carboxyläthenyl)-phenyl]-4,6-dichlorpyrimidin,
2-(4-Phenyläthinylphenyl)-4,6-dichlorpyrimidin,
2-p-Diphenyl-4,6-dichlorpyrimidin,
2-p-Diphenyläther-4,6-dichlorpyrimidin,
2-(4-Pyrrolylphenyl)-4,6-dichlorpyrimidin,
2-(3-Pyrrolylphenyl)-4,6-dichlorpyrimidin,
2-(4-Hydroxypropylaminophenyl)-4,6-dichlorpyrimidin,
2-(4-Dihydroxypropylaminophenyl)-4,6-dichlorpyrimidin.

Als Gegenmittel oder Antidote sind schon verschiedene Stoffe vorgeschlagen worden, welche befähigt sind, die schädigende Wirkung eines Herbizides auf die Kulturpflanze spezifisch zu antagonisieren, d. h. die Kulturpflanze zu schützen, ohne dabei die Herbizidwirkung auf die zu bekämpfenden Unkräuter merklich zu beeinflussen. Dabei kann ein solches Gegenmittel, auch Safener genannt, je nach seinen Eigenschaften, zur Vorbehandlung des Saatgutes der Kulturpflanze (Beizung des Samens oder der Stecklinge) oder vor der Saat in die Saatfurchen oder als Tankmischung zusammen mit dem Herbizid vor oder nach dem Auflaufen der Pflanzen verwendet werden.

So beschreibt die GB-A-1 277 557 die Behandlung von Samen bzw. Sprösslingen von Weizen und Hirse mit gewissen Oxamsäureestern und Amiden vor dem Angriff durch N-Methoxymethyl-2',6'-diäthyl-

chlor-acetanilid (Alachlor). In anderen Literaturstellen (DE-A-1 952 910, DE-A-2 245 471, FR-A-2 021 611) werden Gegenmittel zur Behandlung von Getreide, Mais- und Reis-Samen zum Schutz gegen den Angriff herbizider Thiolcarbamate vorgeschlagen. In der DE-C-1 576 676 und der US-A-3 131 509 werden Hydroxy-amino-acetanilide und Hydantoine für den Schutz von Getreidesamen gegenüber Carbamaten wie IPC, CIPC etc. vorgeschlagen. In der weiteren Entwicklung haben sich alle diese Präparate jedoch als ungenügend erwiesen.

Ueberraschenderweise besitzen Phenylpyrimidine der Formel (I) die Eigenschaft, Kulturpflanzen vor dem Angriff pflanzenaggressiver Agrarchemikalien zu schützen, insbesondere vor Herbiziden der verschiedensten Stoffklassen, wie beispielsweise Chloracetanilide, Triazinone, und Pyridyloxyphenoxypropionsäurederivate, sofern diese nicht oder ungenügend kulturentolerant sind.

Vorzugsweise werden die Kulturpflanzen vor Chloracetanilid, durch die erfindungsgemässen Phenylpyrimidine geschützt.

Ein solches Gegenmittel oder Antidote der Formel (I) kann je nach Anwendungszweck zur Vorbehandlung des Saatgutes der Kulturpflanze (Beizung des Samens oder der Stecklinge) eingesetzt oder vor oder nach der Saat in den Erdboden gegeben werden oder aber für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalie erfolgen. Sie kann jedoch auch gleichzeitig durchgeführt werden (Tankmischung). Vorauflauf-Behandlung schliesst sowohl die Behandlung der Anbaufläche vor der Aussaat (ppi = « pre plant incorporation ») als auch die Behandlung der angesäten, aber noch nicht bewachsenen Anbauflächen ein.

Die Aufwandmengen des Antidotes im Verhältnis zum Herbizid richten sich weitgehend nach der Anwendungsart. Sofern eine Feldbehandlung vorgenommen wird, entweder als Tankmischung oder bei getrennter Applikation von Herbizid und Gegenmittel, verhalten sich die Mengen von Gegenmittel zu Herbizid in der Regel wie 1 : 100 bis 10 : 1, bevorzugt wird jedoch der Bereich 1 : 5 bis 8 : 1, besonders 1 : 1.

Bei Samenbeizung und ähnlich gezielten Schutzmassnahmen werden jedoch weit geringere Mengen Gegenmittel im Vergleich mit den z. B. später pro Hektar Anbaufläche verwendeten Herbizidmengen benötigt. Bei der Samenbeizung werden üblicherweise pro kg Samen 0,1-10 g Gegenmittel benötigt, die bevorzugte Menge liegt zwischen 1 und 2 Gramm. Falls das Gegenmittel kurz vor der Aussaat durch Samenquellen appliziert werden soll, werden z. B. Gegenmittel-Lösungen, welche den Wirkstoff in einer Konzentration von 1-10 000 ppm enthalten, bevorzugt 100-1 000 ppm, verwendet.

In der Regel folgen sich protektive Massnahmen wie Samenbeizung mit einem Gegenmittel der Formel (I) und mögliche spätere Feldbehandlung mit Agrarchemikalien in zeitlich grösserem Abstand. Vorbehandeltes Saat- und Pflanzengut kann später in Landwirtschaft, Gartenbau und Forstwirtschaft mit unterschiedlichen Chemikalien in Berührung kommen. Die Erfindung bezieht sich daher auch auf kulturpflanzenprotektive Mittel, die als Wirkstoff ein Gegenmittel der Formel (I) zusammen mit üblichen Trägerstoffen enthalten. Solche Mittel können gegebenenfalls zusätzlich mit jenen Agrarchemikalien gemischt sein, vor deren Einfluss die Kulturpflanze geschützt werden soll, z. B. mit einem Herbizid der oben genannten Art.

Als Kulturpflanzen gelten im Rahmen vorliegender Erfindung alle Pflanzen, die in irgendeiner Form Ertragsstoffe produzieren (Samen, Wurzeln, Stengel, Knollen, Blätter, Blüten, Inhaltsstoffe wie Oele, Zucker, Stärke, Eiweiss etc.) und zu diesem Zweck angebaut und gehegt werden. Zu diesen Pflanzen gehören beispielsweise sämtliche Getreidearten, Weizen, Roggen, Gerste, Hafer, daneben vor allem Reis, Kulturhirse, Mais, aber auch Baumwolle, Zuckerrüben, Zuckerrohr, Soja, Bohnen, Erbsen.

Das Gegenmittel soll überall dort eingesetzt werden, wo eine Kulturpflanze vor der Phytotoxizität der oben genannten Herbizide geschützt werden soll.

Als Herbizide, vor deren Wirkung es die Kulturpflanzen zu schützen gilt, seien beispielsweise folgende genannt :

Chloracetanilide : 2-Chlor-2',6'-diäthyl-N-(2"-propyloxyäthyl)acetanilid (« Pretilachlor »), 2-Chlor-6'-äthyl-N-(2"-methoxy-1"-methyläthyl)-acet-o-toluidid (« Metolachlor »), 2-Chlor-2',6'-diäthyl-N-(butoxymethyl)acetanilid (« Butachlor »), 2-Chlor-6'-äthyl-N-(äthoxymethyl)acet-o-toluidid (« Acetochlor »), 2-Chlor-6'-äthyl-N-(2"-propoxy-1"-methyläthyl)acet-o-toluidid, 2-Chlor-2',6'-dimethyl-N-(2"-methoxy-1"-methyläthyl)acetanilid, 2-Chlor-2',6'-dimethyl-N-(2"-methoxyäthyl)acetanilid (« Dimethachlor »), 2-Chlor-2',6'-diäthyl-N-(pyrazol-1-ylmethyl)-acetanilid, 2-Chlor-6'-äthyl-N-(pyrazol-1-ylmethyl)-acet-o-toluidid, 2-Chlor-6'-äthyl-N-(3,5-dimethyl-pyrazol-1-ylmethyl)acet-o-toluidid, 2-Chlor-6'-äthyl-N-(2"-butoxy-1"-methyläthyl)acet-o-toluidid (« Metazolachlor »), 2-Chlor-6'-äthyl-N-(2"-butoxy-1"-(methyläthyl) acet-o-zoluidid, 2-Chlor-2'-trimethylsilyl-N-(butoxymethyl)acetanilid, 2-Chlor-2',6'-diäthyl-N-(methoxymethyl)acetanilid (« Alachlor »), 2-Chlor-2',6'-diäthyl-N-(äthoxycarbonylmethyl)acetanilid, 2-Chlor-2',6'-dimethyl-N-(2"-n-propoxyäthyl)acetanilid, 2-Chlor-2',6'-äthyl-6'-methyl-N-(2"-n-propoxyäthyl)acetanilid, 2-Chlor-2',6'-dimethyl-N-(isobutoxymethyl)acetanilid, 2-Chlor-2',6'-dimethyl-N-(isopropoxymethyl)acetanilid oder 2-Chlor-2'-6-tert.butyl-N-(butoxymethyl)acetanilid (« Terbuchlor »).

Triazinone : 4-Amino-6-tert.butyl-4,5-dihydro-3-methylthio-1,2,4-triazin-5-on (« Metribuzin »).

Pyridyloxyphenoxypropionsäure derivate : Propinyl-2-[4'-(3",5"-Dichlorpyridyl-2"-oxy)phenoxy]-propionat (« Chloazifop-propinyl »).

Das 2-Phenyl-, 2-Naphthyl- oder 2-Heterocyclyl-pyrimidin der Formel (I) oder das Mittel, welches dieses Gegenmittel enthält, kann wahlweise vor oder nach der Applikation des Herbizides oder auch gleichzeitig mit diesem angewendet werden. Besonders rationell hat sich das Behandeln der Samen durch eine das Gegenmittel enthaltende Lösung (Samenbeizung) erwiesen. Dabei kann das Lösungsmittel verdunstet werden und der Samen trocken, mit einem Gegenmittel-Belag behaftet zur Anwendung kommen oder man kann den Samen in einer wässerigen, das Gegenmittel enthaltenden Lösung vorquellen und in diesem Zustand aussäen, wie es beispielsweise bei Reis üblich ist.

Die 2-Phenyl-, 2-Naphthyl- oder 2-Heterocyclyl-pyrimidine der Formel (I) können hergestellt werden durch Umsetzen eines entsprechenden Cyanides der Formel (II)

Q—CN (II)

in Methanol mit Natrium-Methylat zum Methoxy-imin der Formel (III)

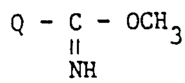

(III)

und weiter mit Ammoniak oder einem Ammoniumsalz weiter zum Amidin der Formel (IV)

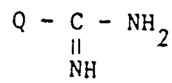

(IV)

welches dann mit einem Malonsäuredialkylester zum 2-Phenyl-, 2-Naphthyl- oder 2-Heterocyclyl-4,6-dihydroxy-pyrimidin der Formel (V) kondensiert wird.

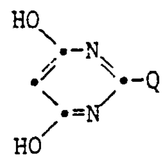

(V)

Dieses wird anschliessend mit einem Halogenierungsmittel zum 2-Phenyl-, 2-Naphthyl- oder 2-Heterocyclyl-pyrimidin der Formel (I) umgesetzt. In den obigen Formeln hat Q die unter Formel (I) gegebene Bedeutung. Diese Umsetzungen gelingen zum grössten Teil bereits bei Raumtemperaturen in organischen polaren Lösungsmitteln. Wo Natriumalkoholat verwendet wird nimmt man den entsprechenden Alkanol als Lösungsmittel, ansonsten eignen sich Ketone, Aether oder aromatische Kohlenwasserstoffe als Lösungsmittel. Die Kondensation des Amidins mit dem Malonsäureester wird vorzugsweise bei der Siedetemperatur des Reaktionsgemisches durchgeführt.

Gemäss einem anderen Herstellungsverfahren kann das Cyanid der Formel (II) in einem aromatischen Kohlenwasserstoff als Lösungsmittel mit Natriumamid direkt zum Amidin der Formel (IV) umgewandelt werden. Dieses wird dann mit einem Malonsäuredialkylester kondensiert.

Gemäss einem weiteren Verfahren kann das Cyanid der Formel (II) mit Salzsäuregas und einem Alkohol in einem inerten Lösungsmittel ins Hydrochlorid des Methoxyimins der Formel (III) und durch weitere Behandlung mit methanolischem Ammoniak ins Hydrochlorid des Amidins der Formel (IV) umgewandelt werden.

Das erfindungsgemässe Verfahren zur Herstellung der 2-Naphthyl- oder 2-Heterocyclylpyrimidine der Formel (I) ist dadurch gekennzeichnet, dass man ein 4,6-Dihydroxy-2-naphthyl- oder -2-heterocyclyl-pyrimidin der Formel (V)

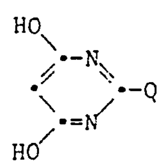

(V)

worin Q die unter Formel I gegebenen Bedeutungen hat, in einem inerten organischen Lösungsmittel mit Halogen oder einem Halogen abgebenden Mittel umsetzt und das erhaltene 4,6-Dihalogen-2-phenyl-, -2-naphthyl- oder -2-heterocyclyl-pyrimidin der Formel (I) isoliert.

Diese Reaktionen werden bei Temperaturen zwischen — 20 °C und dem Siedepunkt des Lösungsmittels vorgenommen, vorzugsweise bei Raumtemperatur. Als Lösungsmittel eignen sich Alkanole, Ketone, Aether, aromatische Kohlenwasserstoffe aber auch z. B. Dimethylsulfoxyd, Dimethylformamid.

Die 2-Phenylpyrimidine der Formel (I) können für sich allein oder zusammen mit den zu antagonisierenden Herbiziden verwendet werden.

Dabei werden Verbindungen der Formel (I) in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel (I) und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle woe epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vir und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate, wie z. B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxyaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi (2-chloräthyl) äthylammoniumbromid.

Die in der Formulierungstechnik gebräuchlichen Tenside sind u.a. in folgenden Publikationen beschrieben :

« Mc Cutcheon's Detergents and Emulsifiers Annual » MC Publishing Corp., Ringwood, New Jersey, 1979. Sisely and Wood, « Encyclopedia of Surface Active Agents », Chemical Publishing Co., Inc. New York, 1964.

Die pestiziden Zubereitungen enthalten in der Regel 0,1 bis 99 %, insbesondere 0,1 bis 95 %, Wirkstoff der Formel (I), 1 bis 99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel, sowie Dünger oder andere Wirkstoffe zur Erzielung spezieller Effekte enthalten.

In den nachfolgenden Beispielen sind die Temperaturen in Celsiusgraden angegeben, Prozente und Angaben von « Teilen » beziehen sich auf das Gewicht.

Beispiel 1

Herstellung von 2-(3,4-Methylendioxyphenyl)-4,6-dichlorpyrimidin

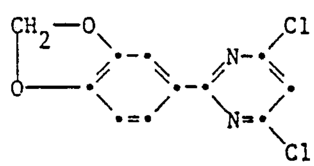

Verbindung No. 143

Man kocht während 3 Stunden am Rückfluss ein Gemisch von 7 g 2-(3,4-Methylendioxyphenyl)-4,6-dihydroxypyrimidin, 6 ml Phosphortrichlorid, 7,6 ml N,N-Dimethylanilin und 30 ml Toluol. Die entstandene Lösung wird schliesslich eingedampft, der Rückstand in Methylenchlorid aufgenommen mit Bleicherde behandelt, über Magnesiumsulfat getrocknet und kristallisiert. Man erhält so 6,3 g Titelprodukt vom Schmelzpunkt 148-150°.

Das als Ausgangsmaterial verwendete 2-(3,4-Methylendioxyphenyl)-4,6-dihydroxy-pyrimidin wird wie folgt hergestellt :

Man sättigt bei 0-5 °C eine Lösung von 25 g 3,4-Methylendioxybenzonitril in 10 ml Methanol und 170 ml Aethylenchlorid mit Salzsäuregas. Die Lösung wird über Nacht bei Raumtemperatur gerührt, dann wird die überschüssige Salzsäure mit Stickstoff ausgetrieben, zur Reaktion 50 ml 10 N Ammoniak in Methanol-Lösung gegeben und das Ganze während 2 Stunden am Rückfluss gekocht und schliesslich am Rotationsverdampfer eingedampft. Der Rückstand wird in 140 ml Methanol gelöst, dann gibt man 26 ml Malonsäurediäthylester und 92 ml 30 %iges Natriummethylat in Methanol dazu und kocht das Gemisch während 6 Stunden am Rückfluss. Das Methanol wird dann abgedampft, der Rückstand in 700 ml Wasser gelöst, filtriert und mit konzentrierter Salzsäure sauer gestellt (pH 1). Der ausgefallene Niederschlag wird abfiltriert, mit Wasser gewaschen und getrocknet. Man erhält so 34,6 g 2-(3,4-Methylendioxyphenyl)-4,6-dihydroxypyrimidin, welches erst bei über 300 °C schmilzt.

Beispiel 2

Herstellung von 2-(4-Benzylideniminophenyl)-4,6-dichlorpyrimidin

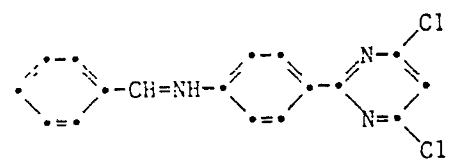

Verbindung No. 139

Eine Lösung von 12 g 2-(4-Aminophenyl)-4,6-dichlorpyrimidin, 6 g Benzaldehyd und 100 mg p-Toluolsulfonsäure werden in 100 ml Toluol während 4 Stunden am Wasserabscheider gekocht. Die Reaktionslösung wird dann bis auf die Hälfte eingedampft, und mit 50 ml Hexan versetzt. Beim Abkühlen kristallisiert aus der Lösung 10,5 g 2-(4-Benzylideniminophenyl)-4,6-dichlorpyrimidin, welches sich bei 190 °C zersetzt.

Beispiel 3

Herstellung von 2-(4-Benzoylamidophenyl)-4,6-dichlorpyrimidin

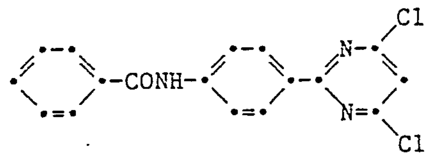

Verbindung No. 149

Zu einer Lösung von 6 g 2-(4-Aminophenyl)-4,6-dichlorpyrimidin, 3 g Triäthylamin und 200 mg 4-Dimethylaminopyridin in 100 ml Tetrahydrofuran werden unter Rühren 3,5 g Benzoylchlorid getropft und nach beendeter Zugabe über Nacht bei Raumtemperatur weitergerührt. Dann giesst man die Reaktionslösung auf Wasser filtriert den erhaltenen Niederschlag, wäscht und trocknet ihn. Man erhält so 8,8 g 2-(4-Benzoylamidophenyl)-4,6-dichlorpyrimidin, welches einen Schmelzpunkt von 198-200 °C aufweist.

Beispiel 4

Herstellung von 2-[4-(Di(2'-Dihydroxyäthyl)amino)-phenyl]-4,6-dichlorpyrimidin

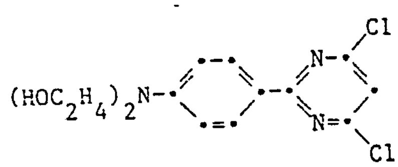

Verbindung No. 148

Man gibt portionenweise unter Rühren bei 0 °C in eine Lösung von 12,5 g Aethylenoxyd in 300 ml Toluol, 48 g 2-(4-Aminophenyl)-4,6-dichlorpyrimidin und 0,5 ml Bor-trifluorid Aetherat. Die entstandene Suspension wird weitergerührt, 30 Minuten bei 5-10 °C, eine Stunde bei Raumtemperatur und schliesslich noch eine Stunde bei 60 °C. Dann wird das Produkt abfiltriert, mit Toluol gewaschen und getrocknet. Man erhält so 33 g Titelprodukt, das aus Tetrahydrofuran/Hexan umkristallisiert wird und bei 169-171 °C schmilzt.

Beispiel 5

Herstellung von 2-[4-(2'-Carboxy-2'-cyanoäthenyl)-phenyl]-4,6-dichlorpyrimidin

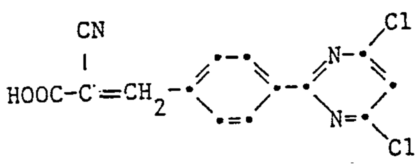

Verbindung No. 135

Eine Lösung von 12,5 g 2-(4-Formylphenyl)-4,6-dichlorpyrimidin, 45 g Cyanessigsäure, 0,2 ml Essigsäure und 0,05 ml Pyrrolidin in 150 ml Toluol wird während einer Stunde am Wasserabscheider gekocht. Dann wird etwas Toluol abdestilliert und die Lösung abgekühlt. Dabei kristallisiert das 2-[4-(2'-Cyano-2-carboxyäthenyl)-phenyl]-4,6-dichlorpyrimidin aus. Man erhält so 15,9 g dieses Produktes, welches bei 240 °C schmilzt.

Beispiel 6

Herstellung von 4,6-Dichloro-2-(1-naphthyl)-pyrimidin

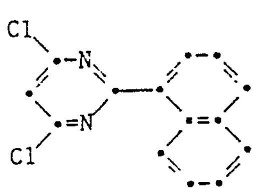

Verbindung No. 1

Man kocht während 2 Stunden am Rückfluss 5 g 4,6-Dihydroxy-2-(1-naphthyl)-pyrimidin, 5 ml N.N-Dimethylanilin und 20 ml Phosphortrichlorid. Die erhaltene Lösung wird dann unter Vakuum am

Rotationsverdampfer eingeengt und der Rückstand auf Eis/Wasser gegossen. Die wässrige Lösung wird mit Aether extrahiert, gewaschen, getrocknet, mit Bleicherde behandelt und eingeengt. Der Rückstand wird aus Aether/Hexan umkristallisiert. Man erhält so 3,1 g kristallines Titelprodukt vom Schmelzpunkt 111-113 °C.

Das als Ausgangsmaterial benötigte 4,6-Dihydroxy-2-(1-naphthyl)-pyrimidin wird wie folgt hergestellt :

Zu 7,3 g 1-Naphthylcyanid in 50 ml Toluol gibt man unter Stickstoffatmosphäre 2,1 ml 50 % Natriumamid in Toluol und rührt über Nacht bei 90 °C. Dann wird die erhaltene Lösung abgekühlt und tropfenweise mit 7,6 ml Malonsäurediäthylester und 13,5 ml 30 % Na-Methylat in Methanol versetzt. Nach beendeter Zugabe wird 5 Stunden unter Rückfluss gekocht. Dann wird abgekühlt, das Reaktionsgemisch mit 100 ml Aether verdünnt und mit 1n wässriger KOH-Lösung extrahiert. Die wässrige Phase wird mit konz. Salzsäure bis pH 2 angesäuert, der ausgefallene Niederschlag wird abfiltriert, mit Wasser gewaschen und im Vakuum bei 100 °C getrocknet. Man erhält so 5 g 4,6-Dihydroxy-2-(1′-naphthyl)-pyrimidin, welches einen Schmelzpunkt von über 300 °C aufweist.

In analoger Weise zu diesen Beispielen werden folgende Verbindungen hergestellt :

Tabelle 1

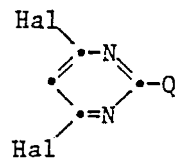

| No. | Hal | | Q | |
|---|---|---|---|---|
| 1 | Cl | Cl | 1-Naphthyl | Smp. 111-113° (Bsp. 6) |
| 2 | Cl | Cl | 5-Methyl-2-thienyl | Smp. 84-87° |
| 3 | Cl | Cl | 5-Chlor-2-thienyl | |
| 4 | Br | Br | 2-Methyl-4-thienyl | Smp. 98-100° |
| 5 | Cl | Cl | 2-Methoxy-4-thienyl | |
| 6 | Br | Br | 2-Thienyl | |
| 7 | Cl | Cl | 2,3-Dimethyl-5-thienyl | |
| 8 | Cl | Cl | 3-Methyl-2-thienyl | |
| 9 | Cl | F | 2-Thienyl | |
| 10 | Cl | Cl | 2-Pyrrolyl | Smp. 85-87° |
| 11 | Cl | Cl | 3-Pyrrolyl | |
| 12 | Cl | Cl | 1-Methyl-2-pyrrolyl | Smp. 80-82° |
| 13 | Cl | Cl | 1-Methyl-3-pyrrolyl | |
| 14 | Cl | Cl | 1-Pyrrolyl | Smp. 58-60° |
| 15 | Cl | Br | 2-Furyl | |
| 16 | Br | Br | 2-Furyl | |
| 17 | Cl | Cl | 5-Methoxy-furyl-(2)- | |
| 18 | Cl | Cl | 5-Methyl-furyl-(2)- | Smp. 91-93° |
| 19 | Cl | Cl | 4-Methyl-furyl-(2)- | |
| 20 | Cl | Cl | 3-Pyrazolyl | |
| 21 | Cl | Cl | 1-Methyl-4-imidazolyl | |
| 22 | Cl | Cl | 1,2,4-Triazol-5-yl | |
| 23 | Cl | Cl | 1,2,3-Triazol-4-yl | |
| 24 | Cl | Cl | 2-Oxthien-4-yl | |
| 25 | Cl | Cl | 1,3-Dithien-4-yl | |
| 26 | Cl | Cl | 1,2-Dithien-4-yl | |
| 27 | Cl | Cl | 1,3-Oxazol-4-yl | |
| 28 | Cl | Cl | 1,2,3-Furazan-4-yl | |
| 29 | Cl | Cl | 1,2,5-Furazan-3-yl | |
| 30 | Cl | Cl | 1,2,4-Furazan-3-yl | |
| 31 | Cl | Cl | 1.3.4-Furazan-2-yl | |
| 32 | Cl | Cl | 1.2.3.4-Oxtriazol-5-yl | |
| 33 | Cl | Cl | 1.2.3.5-Oxtriazol-4-yl | |
| 34 | Cl | Cl | 2.2-Dihydro-1.3.4-dioxazol-5-yl | |
| 35 | Cl | Cl | 1-Methyl-3-pyrazolyl | |
| 36 | Cl | Cl | 1.3.4-Triazol-1-yl | |

Tabelle 1 (Fortsetzung)

| No. | Hal | | Q | |
|---|---|---|---|---|
| 37 | Cl | Cl | 1,2,4-Triazol-4-yl | |
| 38 | Br | Br | 1,2,4-Triazol-4-yl | |
| 39 | Br | Br | 1,3,4-Triazol-4-yl | |
| 40 | F | Cl | 1-Methyl-3-pyrazolyl | |
| 41 | Cl | Cl | 1,3-Oxazol-5-yl | |
| 42 | Cl | Cl | 1,3-Oxazol-4-yl | |
| 43 | Cl | Cl | 1,3-Oxazol-2-yl | |
| 44 | Br | Br | 1,3-Oxazol-2-yl | |
| 45 | Cl | Cl | 1,2-Oxazol-3-yl | |
| 46 | Cl | Cl | 1,3-Thiazol-4-yl | |
| 47 | Cl | Cl | 1,3-Thiazol-5-yl | |
| 48 | Br | Br | 1,3-Thiazol-2-yl | |
| 49 | Cl | Cl | 1,3-Thiazol-2-yl | |
| 50 | Cl | Cl | 2-Methyl-1,3-thiazol-5-yl | Smp. 108-110° |
| 51 | Cl | Cl | 2-Isopropyl-1,3-thiazol-4-yl | |
| 52 | Cl | Cl | 1,2-Thiazol-5-yl | |
| 53 | Br | Br | 2-Pyridyl | |
| 54 | Cl | F | 2-Pyridyl | |
| 55 | Br | Br | 3-Pyridyl | |
| 56 | Br | Br | 4-Pyridyl | |
| 57 | Cl | Cl | 5-Brom-pyrid-3-yl | Smp. 153-154° |
| 58 | Cl | Cl | 2-Methyl-pyrid-4-yl | |
| 59 | Cl | Cl | 3-Methyl-pyrid-5-yl | |
| 60 | Cl | Cl | 2,6-Dimethoxy-pyrid-4-yl | |
| 61 | Cl | Cl | 5-Dimethylamino-pyrid-3-yl | |
| 62 | Cl | Cl | 2-Chlor-pyrid-4-yl | |
| 63 | Cl | Cl | 2-Pyrimidyl | Smp. 150-152° |
| 64 | Cl | Cl | 4,6-Dimethyl-pyrimidin-2-yl | Smp. 178-180° |
| 65 | Br | Br | 4,6-Dimethyl-pyrimidin-2-yl | |
| 66 | Cl | Cl | 2-Chlor-pyrimidin-4-yl | Smp. 117-118° |
| 67 | Cl | Cl | 2-Dimethylamino-pyrimidin-4-yl | Smp. 113-116° |
| 68 | Cl | Cl | 4-Methoxy-6-methyl-pyrimidin-2-yl | |
| 69 | Cl | Cl | 2-Chlor-6-methyl-pyrimidin-2-yl | |
| 70 | Br | Br | 2-Chlor-6-methyl-pyrimidin-2-yl | |
| 71 | Cl | Cl | 2-Äthyl-pyrimidin-5-yl | |
| 72 | Br | Br | 2-Pyrimidinyl | |
| 73 | I | I | 2-Pyrimidinyl | |
| 74 | Cl | Cl | 2-Pyrazinyl | Smp. 138-142° |
| 75 | Cl | Cl | 2-Chlor-pyrazin-5-yl | |
| 76 | Cl | Cl | 3-Pyridazinyl | |
| 77 | Cl | Cl | 6-Methyl-pyridazin-3-yl | |
| 78 | Cl | Cl | 6-Methoxy-pyridazin-3-yl | |
| 79 | Cl | Cl | 6-Chlor-pyridazin-3-yl | Smp. 148-151° |
| 80 | Cl | Cl | 4-Pyridazinyl | |
| 81 | Br | Br | 5-Chlor-pyridazin-3-yl | |
| 82 | Br | Br | 4-Pyridazinyl | |
| 83 | Br | Br | 3-Pyridazinyl | |
| 84 | Cl | Cl | 4,6-Dimethoxy-1,3,5-triazin-2-yl | |
| 85 | Cl | Cl | 4-Äthyl-6-methoxy-1,3,5-triazin-2-yl | |
| 86 | Cl | Cl | 5,6-Dimethyl-1,2,4-triazin-3-yl | |
| 87 | Cl | Cl | 1,2,4-Triazin-3-yl | |
| 88 | Br | Br | 1,2,4-Triazin-3-yl | |
| 89 | Cl | Cl | 3-Methyl-1,2,4-triazin-5-yl | |
| 90 | Cl | Cl | 1,2,3-Triazin-4-yl | |
| 91 | Cl | Cl | 4-Chlor-4-methyl-piperidin-6-yl | |
| 92 | Cl | Cl | 1,4-2H-Oxazin-5-yl | |
| 93 | Cl | Cl | 1,3-6H-Oxazin-2-yl | |
| 94 | Cl | Cl | 1,2,4-Thioxazin-3-yl | |
| 95 | Cl | Cl | 1.2.4-Oxdiazin-3-yl | |
| 96 | Cl | Cl | 2-Benzofuranyl | |

Tabelle 1 (Fortsetzung)

| No. | Hal | | Q | |
|---|---|---|---|---|
| 97 | Cl | Cl | 6-Benzofuranyl | |
| 98 | Cl | Cl | 2-Benzothienyl | |
| 99 | Cl | Cl | 5-Benzothienyl | |
| 100 | Cl | Cl | 5-Benzthiazolyl | |
| 101 | Cl | Cl | 1-Methylindol-3-yl | Smp. 179-181° |
| 102 | Cl | Cl | 3-Indolyl | |
| 103 | Br | Br | 3-Indolyl | |
| 104 | Cl | Cl | 1-Methylindol-5-yl | |
| 105 | Cl | Cl | 1,3-Benzoxazol-2-yl | |
| 106 | Cl | Cl | 1,2-Benzoxazol-5-yl | |
| 107 | Cl | Cl | 6-Isobenzofuranyl | |
| 108 | Cl | Cl | 2-Methylisoindol-5-yl | |
| 109 | Cl | Cl | 2-Methylisoindol-1-yl | |
| 110 | Cl | Cl | 1-Isoindolyl | |
| 111 | Br | Br | 1-Isoindolyl | |
| 112 | F | Cl | 1-Isoindolyl | |
| 113 | Cl | Cl | 6-Indazolyl | |
| 114 | Br | Br | 6-Indazolyl | |
| 115 | Cl | Cl | 3-Chinolyl | Smp. 183-185° |
| 116 | Br | Br | 6-Isochinolyl | |
| 117 | Cl | Cl | 6-Fluorisochinolin-3-yl | |
| 118 | Cl | Cl | 6-Isochinolyl | |
| 119 | Cl | Cl | 6-Chinoxalinyl | Smp. 155° Zers. |
| 120 | Cl | Cl | 2-Methoxy-chinoxalin-7-yl | |
| 121 | Cl | Cl | 2-Methoxy-chinoxalin-6-yl | |
| 122 | Cl | Cl | 4-Chinoxalinyl | |
| 123 | Br | Br | 4-Chinoxalinyl | |
| 124 | Cl | Cl | 2-Chinoxalinyl | |
| 125 | Cl | Cl | 3-Cinnolinyl | |
| 126 | Cl | Cl | 4-Methoxy-chinazolin-2-yl | |
| 127 | Br | Br | 1-Naphthyl | |
| 128 | Cl | Cl | 3-Chromenyl | |
| 129 | Cl | Cl | 3-Chromanyl | |
| 130 | Cl | Cl | 5-Chlor-2-methoxy-pyrid-3-yl | Smp. 166-172° |

Tabelle 2

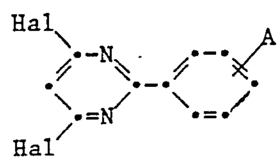

| No. | A | Hal | | Phys. Daten |
|---|---|---|---|---|
| 131 | 4-(β-Phenyläthinyl)- | Cl | Cl | Smp. 143-145° |
| 132 | 3-(β-Phenyläthinyl)- | Cl | Cl | |
| 133 | 4-(2′-Cyanoäthenyl)- | Cl | Cl | |
| 134 | 4-(2′-Carboxyläthenyl)- | Br | Br | |
| 135 | 4-(2′-Carboxyl-2′-cyano-äthenyl)- | Cl | Cl | Smp. 240° Zers. Beispiel 5 |
| 136 | 4-Phenyl- | Cl | Cl | Smp. 111-112° |
| 137 | 4-Phenoxy- | Cl | Cl | Smp. 103-105° |
| 138 | 3-Phenoxy- | Cl | Cl | Smp. 91-93° |
| 139 | 4-Benzylidenamino- | Cl | Cl | Smp. 190° Zers. Beispiel 2 |
| 140 | 3-Benzylidenamino- | Cl | Cl | Smp. 152-154° |
| 141 | 4-Pyrrol-(1)-yl | Cl | Cl | Smp. 119-120° |

Tabelle 2 (Fortsetzung)

| No. | A | Hal | | Phys. Daten |
|---|---|---|---|---|
| 142 | 3-Pyrrol-(1)-yl | Cl | Cl | Smp. 150-151° |
| 143 | 3,4-Methylendioxy- | Cl | Cl | Smp. 148-150° Beispiel 1 |
| 144 | -2,3-Methylendioxy- | Cl | Cl | |
| 145 | 4-(2'-Hydroxyisopropylamino)- | Cl | Cl | Wachs |
| 146 | 4-(2'-Hydroxypropylamino)- | Cl | Cl | Wachs |
| 147 | 4-(2'-Hydroxyäthylamino)- | Cl | Cl | |
| 148 | 4-Di(2'-Hydroxyäthyl)-amino | Cl | Cl | Smp. 169-171° Beispiel 4 |
| 149 | 4-Benzoylamido- | Cl | Cl | Smp. 198-200° Beispiel 3 |
| 150 | 3-(2'-Hydroxyäthylamino)- | Cl | Cl | |
| 151 | 3-(2'-Hydroxy-1'-methylpropylamino)- | Cl | Cl | |
| 152 | 3-(2'-Hydroxy-1'-methylpropylamino)- | Br | Br | |
| 153 | 3-Benzoylamido- | Cl | Cl | |
| 154 | 3-(2'-Cyanoäthenyl)- | Cl | Cl | |

Formulierungsbeispiele

Die Verbindungen der Formel (I) werden im allgemeinen nicht als solche in der Landwirtschaft eingesetzt. Man verwendet gebrauchsfertige formulierte Mittel, welche entweder direkt oder mit Wasser verdünnt eingesetzt werden können.

Die Wirkstoffe werden mit den Trägerstoffen vermischt und vermahlen und können in dieser Form zur Anwendung verstäubt werden.

Beispiel 7
Granulat

Zur Herstellung eines 5 %igen Granulates werden die folgenden Stoffe verwendet :

5 Teile 4,6-Dichlor-2-(1-naphthyl)-pyrimidin oder einer Mischung davon mit 2-Chlor-2',6'-diäthyl.N-(methoxymethyl)-acetanilid,

0,25 Teile epoxidiertes Pflanzenöl,

0,25 Teile Cetylpolyglykoläther,

3,50 Teile Polyäthylenglykol,

91 Teile Kaolin (Korngrösse 0,3-0,8 mm).

Die Aktivsubstanz oder die Mischung wird mit dem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat lässt sich vorteilhaft in Saatfurchen einarbeiten.

Beispiel 8
Emulgierbare Konzentrate

Zur Herstellung eines 25 %igen emulgierbaren Konzentrates werden folgende Stoffe verwendet :

25 Teile 4,6-Dichlor-2-(1-pyrrolyl)-pyrimidin oder einer Mischung davon mit 2-Chlor-6'-äthyl-N-(2"-methoxy-1"-methyläthyl)-acet-o-toluidid,

10 Teile eines Alkylarylsulfonat/Fettalkoholpolyglykoläther-Gemisches,

5 Teile Dimethylformamid,

57,5 Teile Xylol.

Biologische Beispiele

Die Fähigkeit der Verbindungen der Formel (I), Kulturpflanzen vor der phytotoxischen Wirkung starker Herbizide zu schützen, kann aus dem folgenden Beispiel ersehen werden. In den Versuchsbeschreibungen werden die Verbindungen der Formel (I) als Antidote (Gegenmittel) bezeichnet. Die relative Schutzwirkung ist in % angegeben. 0 % bedeutet die Wirkung des Herbizides wenn allein appliziert ; 100 % bedeutet das angestrebte normale Wachstum der Kulturpflanze.

Beispiel 9

Versuch mit Antidote und Herbizid mit in Wasser gesätem Reis. Applikation der Antidote während der Samenquellung des Reises. Reissamen werden während 48 Stunden mit Lösungen der als Antidote zu prüfenden Substanz von 100 ppm getränkt. Anschliessend werden die Samen etwa 2 Stunden trocknen

gelassen, bis sie nicht mehr kleben. Plasrik Container (25 cm lang, 17 cm breit und 12 cm hoch) werden bis 2 cm unter dem Rand mit sandigem Lehm gefüllt. Die vorgequollenen Samen werden auf der Bodenfläche des Containers gesät und nun ganz schwach gedeckt. Die Erde wird in einem feuchten (nicht sumpfigen) Zustand gehalten. Dann wird das Herbizid in verdünnter Lösung auf die Bodenoberfläche versprüht. Der Wasserstand wird entsprechend dem Wachstums sukzessive erhöht. 21 Tage danach wird die realtive Schutzwirkung des Antidots in Prozent bonitiert. Als Referenzen dienen dabei die mit dem Herbizid allein behandelten Pflanzen (keine Schutzwirkung) sowie die vollständig unbehandelte Kontrolle (100 % Wachstum). Die Ergebnisse sind in der untenstehenden Tabelle zusammengefasst.

Als Herbizid wird 2-Chlor-2,6-diäthyl-N-(2″-propyloxyäthyl)-acetanilid (« Pretilachlor ») in einer Aufwandmenge von 0,25 kg pro Hektar verwendet.

| Verbindung No. | relative Schutzwirkung % | Verbindung No. | relative Schutzwirkung % |
|---|---|---|---|
| 1 | 38 | 115 | 38 |
| 2 | 63 | 130 | 12.5 |
| 4 | 38 | 131 | 12.5 |
| 10 | 63 | 136 | 25 |
| 12 | 50 | 137 | 38 |
| 18 | 50 | 139 | 50 |
| 50 | 63 | 140 | 63 |
| 57 | 25 | 141 | 25 |
| 63 | 25 | 142 | 12.5 |
| 64 | 25 | 143 | 38 |
| 66 | 12.5 | 145 | 38 |
| 67 | 12.5 | 147 | 38 |
| 74 | 38 | 148 | 12.5 |
| 79 | 63 | | |

Beispiel 10

Versuch mit Antidote und Herbizid in Reis. Applikation von Antidote und Herbizid als Tankmischung im Vorauflaufverfahren

Reissamen werden während 48 Stunden in Wasser vorgequollen. Plastik-Container (25 cm lang, 17 cm breit und 12 cm hoch) werden mit Erde gefüllt, in die die vorgequollenen Reissamen eingesät werden. Anschliessend wird die als Antidote zu prüfende Substanz zusammen mit dem Herbizid als Tankmischung versprüht. Der Wasserstand wird entsprechend dem Wachstum der Reispflanzen sukzessive erhöht. 18 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100 % relative Schutzwirkung).

Die Resultate sind untenstehend zusammengefasst.

Herbizid : 2-Chlor-2′,6′-diäthyl-N-(2″-propyloxyäthyl)-acetanilid (« Pretilachlor »)

| Antidote Verbindung No. | Aufwandmenge kg/ha | Herbizid Aufwandmenge kg/ha | relative Schutzwirkung in % |
|---|---|---|---|
| 2 | 1 | 1 | 25 |
| 2 | 0,5 | 0,5 | 75 |
| 10 | 1 | 1 | 38 |
| 10 | 0,5 | 0,5 | 63 |
| 12 | 1 | 1 | 38 |
| 12 | 0,5 | 0,5 | 63 |
| 74 | 1 | 1 | 63 |
| 74 | 0,5 | 0,5 | 75 |
| 143 | 1 | 1 | 38 |
| 143 | 0,5 | 0,5 | 38 |

Beispiel 11

Versuch mit Antidote und Herbizid in verpflanztem Reis. Applikation von Antidote und Herbizid als Tankmischung im Verauflaufverfahren

Reispflanzen werden bis zum 1 1/2-2 Blattstadium in Erde aufgezogen. Die Pflanzen werden dann büschelweise (immer 3 Pflanzen zusammen) in Container (47 cm lang, 29 cm breit und 24 cm hoch) in sandigen Lehm verpflanzt. Die Bodenoberfläche wird anschliessend mit Wasser von 1,5-2 cm Höhe beschichtet. 2-3 Tage nach dem Verpflanzen wird das Herbizid zusammen mit der als Antidote zu prüfenden Substanz als Tankmischung direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100 % Schutzwirkung).

Die Resultate sind wie folgt :

Verbindung No. 1

Herbizid : 2-Chlor-2',6'-diäthyl-N-(2-propyloxyäthyl)-acetanilid (« Pretilachlor »)

| Antidote Verbindung No. | Aufwandmenge kg/ha | Herbizid Aufwandmenge kg/ha | relative Schutzwirkung in % |
|---|---|---|---|
| 143 | 1 | 1 | 12.5 |
| 143 | 0,5 | 1 | 12.5 |
| 143 | 0,75 | 0,75 | 50 |
| 143 | 0,375 | 0,75 | 25 |

Beispiel 12

Versuch mit Antidote und Herbizid in trocken gesätem Reis. Applikation des Antidotes als Samenbeize

Reissamen werden mit der als Safener zu prüfenden Substanz in einen Glasbehälter gemischt. Samen und Produkt werden durch Schütteln und Rotation gut zusammengemischt. Dann werden Container (47 cm lang, 29 cm breit und 24 cm hoch) mit sandiger Erde gefüllt und die gebeizten Samen werden eingesät. Nach dem Bedecken des Samens wird das Herbizid in einer verdünnten Lösung auf die Bodenoberfläche versprüht. Etwa 20 Tage nach der Saat (3-Blattstadium der Reispflanzen) wird die Bodenoberfläche mit 4 cm Wasser Höhe beschichtet. 30 Tage nach der Herbizidapplikation wird die Schutzwirkung des Safeners in Prozent bonitiert. Als Referenzen dienen dabei die mit Herbizid allein behandelten Pflanzen (keine Schutzwirkung) sowie die vollständig unbehandelte Kontrolle (= 100 % relative Schutzwirkung). Die Resultate sind wie folgt :

Herbizid : 2-Chlor-2'-äthyl-6-methyl-N-(2"-methoxy-1"-methyläthyl)-acetanilid (« Metolachlor »)

| Antidote Verbindung No. | Aufwandmenge kg/ha | Herbizid Aufwandmenge kg/ha | relative Schutzwirkung in % |
|---|---|---|---|
| 2 | 0,5 | 0,5 | 25 |
| 2 | 0,5 | 0,25 | 25 |
| 10 | 0,5 | 0,5 | 25 |
| 10 | 0,5 | 0,25 | 12.5 |
| 12 | 0,5 | 0,5 | 25 |
| 12 | 0,5 | 0,25 | 38 |
| 143 | 0,5 | 0,5 | 25 |
| 143 | 0,5 | 0,25 | 38 |

Beispiel 13

Versuch mit Antidote und Herbizid in Reis. Applikation des Antidotes als Samenbeize

Reissamen werden mit der als Safener zu prüfenden Substanz in einen Glasbehälter gemischt. Samen und Produkt werden durch Schütteln und Rotation gut zusammengemischt. Anschliessend werden Plastikcontainer (47 cm lang, 29 cm breit und 24 cm hoch) mit sandiger Lehmerde gefüllt und die gebeizten Samen werden eingesät. Nach dem Bedecken des Samens wird das Herbizid auf die

Bodenoberfläche versprüht. 18 Tage nach der Saat wird die Schutzwirkung des Safeners in Prozent bonitiert. Als Referenzen dienen dabei die mit Herbizid behandelten Pflanzen (keine Schutzwirkung) sowie die vollständig unbehandelte Kontrolle (= 100 % relative Schutzwirkung).

Die Resultate sind wie folgt :

Herbizid : 2-Chlor-2'-Aethyl-6'-methyl-N-(1'-methoxy-1'-methyläthyl)-acetanilid (« Metolachlor »)

| Antidote Verbindung Nr | Aufwandmenge k/kg Samen | Herbizid Aufwandmenge kg/ha | relative Schutzwirkung in % |
|---|---|---|---|
| 2 | 0,5 | 0,5 | 25 |
| 2 | 0,5 | 0,25 | 38 |
| 10 | 0,5 | 0,5 | 25 |
| 10 | 0,5 | 0,25 | 12.5 |
| 12 | 0,5 | 0,5 | 25 |
| 12 | 0,5 | 0,25 | 38 |

Beispiel 14

Versuch mit Antidote und Herbizid in Sorghum (Hirse). Applikation von Herbizid und Antidote als Tankmischung im Vorauflaufverfahren

Töpfe, welche einen oberen Durchmesser von 6 cm haben, werden mit sandiger Lehmerde gefüllt und Sorghumsamen der Sorte G522 werden eingesät. Nach dem Bedecken der Samen wird die als Safener zuprüfende Substanz zusammen mit dem Herbizid in verdünnter Lösung als Tankmischung auf die Bodenoberfläche versprüht. 21 Tage nach der Herbizidapplikation wird die Schutzwirkung des Safeners in Prozent bonitiert. Als Referenzen dienen dabei die mit Herbizid allein behandelten Pflanzen (keine Schutzwirkung) sowie die vollständig unbehandelte Kontrolle (= 100 % relative Schutzwirkung.

Die Resultate sind wie folgt :

Herbizid : 2-Chlor-2'-äthyl-6'-methyl-N-(2''-methoxy-2''-methyläthyl)-acetanilid (« Metolachlor »)

| Antidote Verbindung No. | Aufwandmenge kg/ha | Herbizid Aufwandmenge kg/ha | relative Schutzwirkung in % |
|---|---|---|---|
| 115 | 1,5 | 1,5 | 25 |
| 135 | 1,5 | 1,5 | 50 |

Beispiel 15

Versuch mit Antidote und Herbizid in Weizen. Applikation von Antidote und Herbizid als Tenkmix im Nachauflaufverfahren

Weizensamen der Sorte « Farnese » werden in Plastiktöpfe (oberer Durchmesser 11 cm), die 0,5 l Erde enthalten, im Gewächshaus ausgesät. Nach dem Bedecken der Samen wird die als Safener zu prüfende Substanz zusammen mit dem Herbizid als Tankmischung im Nachauflaufverfahren appliziert. 20 Tage nach der Applikation wird die Schutzwirkung des Safeners in Prozent bonitiert. Als Referenzen dienen dabei die mit Herbizid allein behandelten Pflanzen (keine Schutzwirkung) sowie die vollständig unbehandelte Kontrolle (= 100 % relative Schutzwirkung).

Die Resultate sind wie folgt :

Herbizid : α-[3-(2,4-Dichlorpyridyl-2-oxy)-phenoxy]-propionsäurepropinylester (« Chlorazifof-propinyl »)

| Antidote Verbindung No. | Aufwandmenge kg/ha | Herbizid Aufwandmenge kg/ha | relative Schutzwirkung in % |
|---|---|---|---|
| 2 | 1,5 | 0,75 | 25 |
| 50 | 1,5 | 0,75 | 63 |
| 79 | 1,5 | 0,75 | 63 |
| 115 | 1,5 | 0,75 | 63 |
| 141 | 1,5 | 0,75 | 50 |
| 149 | 1,5 | 0,75 | 25 |

Beispiel 16

Versuch mit Antidote und Herbizid in Soja. Applikation von Antidote und Herbizid als Tankmix im Verauflaufverfahren

Plastikcontainer (25 cm lang, 17 cm breit und 12 cm hoch) werden mit sandiger Lehmerde gefüllt und Sojasamen der Sorte « HARK » eingesät. Nach dem Nedecken der Samen werden die als Safener zu prüfende Substanz zusammen mit dem Herbizid in verdünnte Lösung als Tankmischung auf die Bodenoberfläche gesprüht. 30 Tage nach der Applikation wird die Schutzwirkung des Safeners in Prozent bonitiert. Als Referenzen dienen dabei die mit dem Herbizid allein behandelten Pflanzen (keine Schutzwirkung) sowie die vollständig unbehandelte Kontrolle (= 100 % relative Schutzwirkung). Das Resultat ist wie folgt :

Herbizid : 4-Amino-4,5-dihydro-3-methylthio-1,2,4-triazin-5-on (Metribuzin)

| Antidote Verbindung No. | Aufwandmenge kg/ha | Herbizid Aufwandmenge kg/ha | relative Schutzwirkung in % |
|---|---|---|---|
| 137 | 1,5 | 0,75 | 25 |

**Patentansprüche**

1. Mittel zum Schützen von Kulturpflanzen vor Schädigung durch Herbizide, dadurch gekennzeichnet, dass es neben inerten Träger- und Zusatzstoffen als wirksame Komponente ein 2-Phenyl-, 2-Naphthyl- oder 2-Heterocyclyl-pyrimidin der Formel (I) enthält,

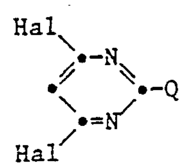

(I)

worin Hal ein Halogenatom,
Q einen Phenylrest

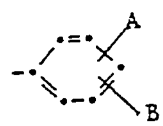

einen unsubstituierten α-Naphthylrest, oder einen ungesättigten oder teilweise gesättigten oder auch benzannelierten 5-6 gliedrigen Heterocyclus, der unsubstituiert ist oder ein- oder mehrmals durch Halogen, $C_1$-$C_6$-Alkyl, $C_1$-$C_6$-Alkoxy oder Dimethylamino substituiert ist und worin im Phenylrest Q A einen Rest Phenyl, Phenoxy, Phenyl-$C_{2-6}$ alkinyl, Benzylidenimino, Benzoylamido, Pyrrol-1-yl oder einen $C_2$-$C_6$-Alkenylrest, der durch Cyan und/oder Carboxy substituiert ist oder einen durch ($C_1$-$C_4$-Hydroxyalkyl) mono oder disubstituierten A aminorest, und
B Wasserstoff oder
A und B zusammen die Methylendioxygruppe —O—$CH_2$—O bedeuten, mit der Massgabe, dass wenn Hal Chlor bedeutet, Q nicht ein unsubstituierter Pyridyl-, Furyl- oder Thienylrest oder ein 2-Dimethylamino-4-pyrimidinylrest sein kann.

2. Die 2-Naphthyl- oder 2-Heterocyclyl-pyrimidine der Formel (I)

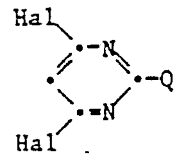

(I)

worin Hal Chlor oder Brom,
Q einen unsubstituierten α-Naphthylrest oder einen ungesättigten oder teilweise gesättigten und/oder benzannelierten 5-6 gliedrigen Heterocyclus, der unsubstituiert ist oder der ein- oder mehrmals durch

Halogen, $C_1$-$C_6$-Alkyl, $C_1$-$C_6$-Alkoxy oder Dimethylamino substituiert ist, mit der Massgabe, dass wenn Hal Chlor bedeutet, Q nicht ein unsubstituierter Pyridyl-, Furyl- oder Thienyl- oder ein 2-Dimethylamino-4-pyrimidinylrest sein kann.

3. Phenylpyrimidine der Formel (Ia)

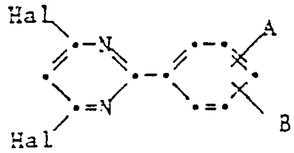

(Ia)

worin Hal Chlor oder Brom,
A einen Rest Phenyl, Phenoxy, Phenyl-$C_2$-$C_6$-alkinyl, Benzylidenimino, Benzoylamido, Pyrrol-1-yl, einen $C_2$-$C_6$-Alkenylrest, der durch Cyan und/oder Carboxyl substituiert ist ; einen $C_1$-$C_4$-Hydroxy alkylaminorest und
B Wasserstoff oder
A und B zusammen die Methylendioxygruppe $—O—CH_2—O—$ bedeuten.

4. Die Phenylpyrimidine der Formel (Ia), Anspruch 3, worin A einen Rest Phenyl oder Phenoxy und B Wasserstoff bedeutet.

5. Die Phenylpyrimidine der Formel (Ia), Anspruch 3, worin A ein durch Cyan oder COOH substituiertes $C_2$-$C_6$ Alkenyl oder ein durch Phenyl substituiertes $C_2$-$C_6$ Alkinyl und B Wasserstoff bedeutet.

6. Die Phenylpyrimidine der Formel (Ia), Anspruch 3, worin A einen Rest $—NHC_2H_4OH—NHCH(CH_3)CH_2OH$ oder $N(C_2H_4OH)_2$ und B Wasserstoff bedeutet.

7. Die Phenylpyrimidine der Formel (Ia), Anspruch 3, in denen A und B zusammen die Methylendioxy-Kette bilden.

8. Die Phenylpyrimidine der Formel (Ia), Anspruch 3, worin A einen unsubstituierten über Stickstoff gebundenen Pyrrolylrest und B Wasserstoff bedeutet.

9. Ein Mittel gemäss Anspruch 1, das als wirksame Komponente ein 2-Heterocyclylpyrimidin der Formel (I), Anspruch 1, enthält in dem Hal Chlor oder Brom und Q einen ungesättigten oder teilweise gesättigten oder auch benzannelierten 5-6 gliedrigen Heterocyclus bildet, der unsubstituiert oder ein- oder mehrfach durch Halogen, $C_1$-$C_6$ Alkyl, $C_1$-$C_6$ Alkoxy oder Dimethylamino substituiert ist, mit der Massgabe, dass, wenn Hal je Chlor ist, Q nicht einen unsubstituierten Pyridyl-, Furyl- oder Thienylrest und auch nicht den 2-Dimethylamino-4-pyrimidinylrest sein darf.

10. Ein Mittel gemäss Anspruch 1 das als wirksame Komponente ein unsubstituiertes 4,6-Dihalo-2-(α-naphthyl)-pyrimidin enthält, worin Hal Chlor oder Brom bedeutet.

11. Die 2-Heterocyclylpyrimidine der Formel (I), Anspruch 2, in denen Q einen Furylrest bedeutet, der durch Methyl, Methoxy substituiert ist, während Hal Chlor oder Brom bedeutet.

12. 4,6-Dichlor-2-(1′-naphthyl)-pyrimidin gemäss Ansprüch 2.

13. 4,6-Dichlor-2-(2′-methyl-5′-thienyl)-pyrimidin gemäss Anspruch 2.

14. 4,6-Dichlor-2-(1′-pyrrol)-pyrimidin gemäss Anspruch 3.

15. 4,6-Dichlor-2-(1-methyl-pyrrol-2′-yl)-pyrimidin gemäss Anspruch 2.

16. 4,6-Dichlor-2-(5′-brom-pyrid-4-yl)-pyrimidin gemäss Anspruch 2.

17. 4,6-Dichlor-2-(2′-pyrimidinyl)-pyrimidin gemäss Anspruch 2.

18. Mittel gemäss Anspruch 1, welches als Wirkstoff 4,6-Dichlor-2-(4′,6′-dimethylpyrimidin-2-yl)-pyrimidin enthält.

19. 2-(3,4-Methylendioxyphenyl)-4,6-dichlorpyrimidin gemäss Anspruch 3.

20. 2-(4-Benzylidenaminophenyl)-4,6-dichlorpyrimidin gemäss Anspruch 3.

21. 2-[4-Di(2′-hydroxyäthyl) aminophenyl]-4,6-dichlorpyrimidin gemäss Anspruch 3.

22. 2-(4-Phenyläthinylphenyl)-4,6-dichlorpyrimidin gemäss Anspruch 3.

23. 2-Biphenyl-4,6-dichlorpyrimidin gemäss Anspruch 3.

24. 2-(4-Diphenyläther)-4,6-dichlorpyrimidin gemäss Anspruch 3.

25. 2-(4-Pyrrolylphenyl)-4,6-dichlorpyrimidin gemäss Anspruch 3.

26. 2-(3-Pyrrolylphenyl)-4,6-dichlorpyrimidin gemäss Anspruch 3.

27. Verfahren zur Herstellung der 2-Phenyl-, 2-Naphthyl- und 2-Heterocyclylpyrimidine der Formel (I), Anspruch 1, dadurch gekennzeichnet, dass man ein 4,6-Dihydroxy-2-naphthyl- oder 2-heterocyclyl-pyrimidin der Formel (V)

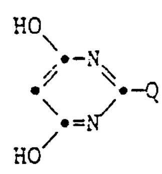

(V)

worin Q die im Anspruch 1 gegebene Bedeutung hat, in einem inerten organischen Lösungsmittel mit Halogen oder einem Halogen abgebenenden Mittel behandelt, und das erhaltene 4,6-Dihalogen-2-phenyl-2-naphthyl- oder 2-heterocyclyl-pyrimidin der Formel (I) isoliert.

28. Verwendung der 2-Phenyl-, 2-Naphthyl- und 2-Heterocyclyl-pyrimidine der Formel (I) gemäss Anspruch 1 zum Schützen von Kulturpflanzen gegen die schädigende Wirkung von Chloracetanilid-, Pyridyloxyphenoxypropionsäure- oder Triazinon-Herbiziden.

29. Verwendung der 2-Phenyl-, 2-Naphthyl- und 2-Heterocyclyl-pyrimidine der Formel (I) gemäss Anspruch 1, zum Schützen von Getreide, Reis-, Mais-, Sorghum- und Soja-Kulturen vor der phytotoxischen Schädigung durch Chloracetanilid-, Pyridyloxyphenoxypropionsäure- oder Triazinon-Herbizide.

30. Verfahren zum Schützen von Kulturpflanzen vor Schäden, die bei der Applikation von Chloracetanilid-, Pyridyloxyphenoxypropionsäure- oder Triazinon-Herbiziden auftreten, dadurch gekennzeichnet, dass man

a) die Anbaufläche für die Pflanze vor oder während der Applikation des Herbizids oder

b) den Samen oder die Stecklinge der Pflanzen oder die Pflanze selbst mit einer wirksamen Menge eines 2-Phenyl-, 2-Naphthyl- oder 2-Heterocyclyl-pyrimidins der Formel (I) gemäss Anspruch 1 behandelt.

31. Verfahren gemäss den Ansprüchen 28-30 zum Schützen von Reis vor der Schädigung durch Chloracetanilid-Herbizide.

32. Saatgut von Kulturpflanzen, welches mit einer wirksamen Menge eines 2-Phenyl-, 2-Naphthyl- oder 2-Heterocyclyl-pyrimidins der Formel (I), Anspruch 1, behandelt wurde.

**Claims**

1. A composition for protecting cultivated plants from damage by herbicides, which composition contains as active component, together with inert carriers and adjuvants, a 2-phenylpyrimidine, 2-naphthylpyrimidine or 2-heterocyclylpyrimidine of the formula (I)

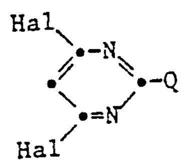

(I)

in which Hal is a halogen atom,
Q is a phenyl radical

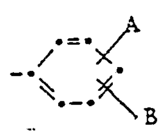

an unsubstituted α-naphthyl radical or an unsaturated or partially saturated or also benzofused 5- or 6-membered heterocycle that is unsubstituted or mono- or poly-substituted by halogen, $C_1$-$C_6$ alkyl, $C_1$-$C_6$ alkoxy or by dimethylamino, and
wherein in the phenyl radical Q A is a phenyl, phenoxy, phenyl-$C_{2-6}$ alkynyl, benzylideneimino, benzoylamido, pyrrol-1-yl or $C_2$-$C_6$ alkenyl radical that is substituted by cyano and/or carboxy, or is an amino radical that is mono- or disubstituted by ($C_1$-$C_4$ hydroxyalkyl), and
B is hydrogen or
A and B together are the methylenedioxy group —O—$CH_2$—O, with the proviso that when Hal is chlorine Q cannot be an unsubstituted pyridyl, furyl or thienyl radical or a 2-dimethylamino-4-pyrimidinyl radical.

2. The 2-naphthylpyrimidines or 2-heterocyclylpyrimidines of the formula (I)

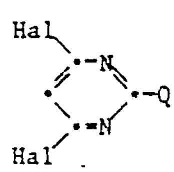

(I)

in which Hal is chlorine or bromine,
Q is an unsubstituted α-naphthyl radical or an unsaturated or partially saturated and/or benzofused 5- or 6-membered heterocycle that is unsubstituted or mono- or poly-substituted by halogen, $C_1$-$C_6$ alkyl, $C_1$-$C_6$ alkoxy or by dimethylamino, with the proviso that when Hal is chlorine Q cannot be an unsubstituted pyridyl, furyl or thienyl radical or a 2-dimethylamino-4-pyrimidinyl radical.

3. Phenylpyrimidines of the formula (Ia)

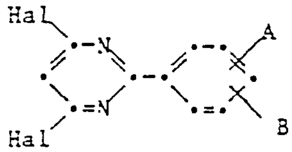

wherein Hal is chlorine or bromine,
A is a phenyl, phenoxy, phenyl-$C_2$-$C_6$ alkynyl, benzylideneimino, benzoylamido, pyrrol-1-yl or $C_2$-$C_6$ alkenyl radical that is substituted by cyano and/or carboxy, or a $C_1$-$C_4$-hydroxyalkylamino radical and B is hydrogen or
A and B together are the methylenedioxy group $—O—CH_2—O$.

4. The phenylpyrimidines of the formula (Ia), claim 3, wherein A is a phenyl or phenoxy radical and B is hydrogen.

5. The phenylpyrimidines of the formula (Ia), claim 3, wherein A is a $C_2$-$C_6$ alkenyl radical substituted by cyano or COOH, or is a $C_2$-$C_6$ alkynyl radical substituted by phenyl, and B is hydrogen.

6. The phenylpyrimidines of the formula (Ia), claim 3, wherein A is an $—NHC_2H_4OH$, $—NHCH(CH_3)CH_2OH$ or $N(C_2H_4OH)_2$ radical and B is hydrogen.

7. The phenylpyrimidines of the formula (Ia), claim 3, wherein A and B together form the methylenedioxy chain.

8. The phenylpyrimidines of the formula (Ia), claim 3, wherein A is an unsubstituted pyrrolyl radical bonded by way of nitrogen and B is hydrogen.

9. A composition according to claim 1 that contains as active component a 2-heterocyclylpyrimidine of the formula (I), claim 1, in which Hal is chlorine or bromine and Q is an unsaturated or partially saturated or also benzofused 5- or 6-membered heterocycle that is unsubstituted or mono- or poly-substituted by halogen, $C_1$-$C_6$ alkyl, $C_1$-$C_6$ alkoxy or by dimethylamino, with the proviso that when Hal is chlorine Q may not be an unsubstituted pyridyl, furyl or thienyl radical or a 2-dimethylamino-4-pyrimidinyl radical.

10. A composition according to claim 1 that contains as active component an unsubstituted 4,6-dihalo-2-(α-naphthyl)-pyrimidine wherein Hal is chlorine or bromine.

11. The 2-heterocyclylpyrimidines of the formula (I), claim 2, in which Q is a furyl radical that is substituted by methyl or methoxy and Hal is chlorine or bromine.

12. 4,6-Dichloro-2-(1′-naphthyl)-pyrimidine according to claim 2.

13. 4,6-Dichloro-2-(2′-methyl-5′-thienyl)-pyrimidine according to claim 2.

14. 4,6-Dichloro-2-(1′-pyrrole)-pyrimidine according to claim 3.

15. 4,6-Dichloro-2-(1-methyl-pyrrol-2′-yl)-pyrimidine according to claim 2.

16. 4,6-Dichloro-2-(5′-bromo-pyrid-4-yl)-pyrimidine according to claim 2.

17. 4,6-Dichloro-2-(2′-pyrimidinyl)-pyrimidine according to claim 2.

18. A composition according to claim 1 that contains as active ingredient 4,6-dichloro-2-(4′,6′-dimethylpyrimidin-2-yl)-pyrimidine.

19. 2-(3,4-Methylenedioxyphenyl)-4,6-dichloropyrimidine according to claim 3.

20. 2-(4-Benzylideneaminophenyl)-4,6-dichloropyrimidine according to claim 3.

21. 2-[4-Di(2′-hydroxyethyl) aminophenyl]-4,6-dichloropyrimidine according to claim 3.

22. 2-(4-Phenylethynylphenyl)-4,6-dichloropyrimidine according to claim 3.

23. 2-Biphenyl-4,6-dichloropyrimidine according to claim 3.

24. 2-(4-Diphenyl ether)-4,6-dichloropyrimidine according to claim 3.

25. 2-(4-Pyrrolylphenyl)-4,6-dichloropyrimidine according to claim 3.

26. 2-(3-Pyrrolylphenyl)-4,6-dichloropyrimidine according to claim 3.

27. A process for the preparation of 2-phenylpyrimidines, 2-naphthylpyrimidines and 2-heterocyclylpyrimidines of the formula (I), claim 1, which comprises treating a 4,6-dihydroxy-2-naphthylpyrimidine or 2-heterocyclylpyrimidine of the formula (V)

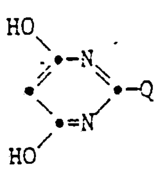

(V)

in which Q has the meaning given in claim 1, with halogen or a halogen-yielding agent in an inert organic solvent, and isolating the resulting 4,6-dihalo-2-phenyl-2-naphthylpyrimidine or 2-heterocyclylpyrimidine of the formula (I).

28. Use of the 2-phenylpyrimidines, 2-naphthylpyrimidines and 2-heterocyclylpyrimidines of the formula (I) according to claim 1 for protecting cultivated plants from the damaging action of chloroacetanilide, pyridyloxyphenoxypropionic acid or triazinone herbicides.

29. Use of the 2-phenylpyrimidines, 2-naphthylpyrimidines and 2-heterocyclylpyrimidines of the formula (I) according to claim 1 for protecting cereals, rice, maize, sorghum and soybean crops from the phytotoxic damage caused by chloroacetanilide, pyridyloxyphenoxypropionic acid or triazinone herbicides.

30. A method of protecting cultivated plants from damage that occurs as a result of the application of chloroacetanilide, pyridyloxyphenoxypropionic acid or triazinone herbicides, which comprises

a) treating the locus of the plant before or during application of the herbicide, or

b) treating the seeds or cuttings of the plant or the plant itself with an effective amount of a 2-phenylpyrimidine, 2-naphthylpyrimidine or 2-heterocyclylpyrimidine of the formula (I) according to claim 1.

31. A method according to claims 28 to 30 of protecting rice from damage by chloroacetanilide herbicides.

32. Seeds of cultivated plants that have been treated with an effective amount of a 2-phenylpyrimidine, 2-naphthylpyrimidine or 2-heterocyclylpyrimidine of the formula (I), claim 1.

## Revendications

1. Moyen pour protéger les plantes cultivées des dégâts causés par des herbicides, caractérisé en ce qu'outre des véhicules et additifs inertes il contient comme composant actif une 2-phényl-, 2-naphtyl- ou 2-hétérocyclyl-pyrimidine de formule (I)

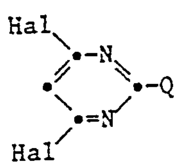
(I)

dans laquelle Hal est un atome d'halogène,
Q est un reste phényl

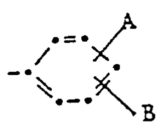

un reste non substitué α-naphtyle ou un hétérocycle insaturé ou partiellement saturé ou un hétérocycle à 5-6 membres à caractère benzénique, qui est non substitué ou substitué une ou plusieurs fois par un halogène, alkyle $C_1$-$C_6$, alcoxy $C_1$-$C_6$ ou diméthylamino et dans laquelle dans le reste phényle Q A est un reste phényle, phénoxy, phényl-alcynyle $C_2$-$C_6$, benzylidèneimino, benzoylamido, pyrrolyl-1 ou un reste alcényle $C_2$-$C_6$, qui est substitué par un cyano ou un carboxy, ou un reste hydroxyalkylamino $C_1$-$C_4$, et B est l'hydrogène ou
A et B représentent ensemble le groupe méthylènedioxy —O—$CH_2$—O, à la condition que, lorsque Hal représente le chlore, Q ne peut pas être un reste pyridyle, furyle ou thiényle non substitué ou un reste 2-diméthylamino-4-pyrimidinyle.

2. 2-naphtyl- ou 2-hétérocyclyl-pyrimidine de formule (I)

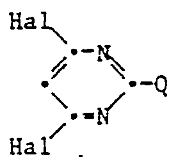
(I)

dans laquelle Hal est le chlore ou le brome,
Q est un reste α-naphtyle non substitué ou un hétérocycle insaturé ou partiellement saturé et/ou un hétérocycle à 5-6 membres à caractère benzénique, qui est non substitué ou substitué une ou plusieurs fois par un halogène, alkyle $C_1$-$C_6$, alcoxy $C_1$-$C_6$ ou diméthylamino, avec la condition que, quand Hal représente le chlore, Q ne peut pas être un reste pyridyle, furyle ou thiényle non substitué ou un reste 2-diméthylamino-4-pyrimidinyle.

3. Phénylpyrimidine de formule (Ia)

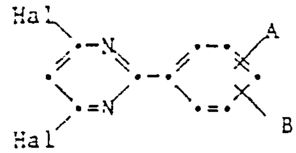

dans laquelle Hal est le chlore ou le brome, A est un reste phényle, phénoxy, phénylalcynyle $C_2$-$C_6$, benzylidène-imino, benzoylamido, pyrrolyl-1, un reste alcényle $C_2$-$C_6$ qui est substitué par un cyano et ou un carboxyle ; un reste hydroxyalkylamino $C_1$-$C_4$ et B est l'hydrogène ou A et B représentent ensemble le groupe méthylènedioxy —O—$CH_2$—O—.

4. Phénylpyrimidines de formule (Ia) selon la revendication 3, dans lesquelles A est un reste phényle ou phénoxy et B est l'hydrogène.

5. Phénylpyrimidines de formule (Ia) selon la revendication 3, dans lesquelles A est un alcényle $C_2$-$C_6$ substitué par un cyano ou COOH ou un alcynyle $C_2$-$C_6$ substitué par un phényle et B est l'hydrogène.

6. Phénylpyrimidines de formule (Ia) selon la revendication 3, dans lesquelles A est un reste —$NHC_2H_4OH$, —$NHCH(CH_3)CH_2OH$ ou $N(C_2H_4OH)_2$ et B est l'hydrogène.

7. Phénylpyrimidines de formule Ia, selon la revendication 3, dans lesquelles A et B ensemble forment la chaîne méthylènedioxy.

8. Phénylpyrimidines de formule (Ia) selon la revendication 3 dans laquelle A est un reste pyrrolyle non substitué lié par l'azote et B est l'hydrogène.

9. Moyen selon la revendication 1 qui contient comme composant actif une 2-hétérocyclylpyrimidine de formule (I) selon la revendication (I), dans laquelle Hal est le chlore ou le brome et Q forme un hétérocycle non saturé ou partiellement saturé ou aussi un hétérocycle à 5-6 membres à caractère benzénique, qui est non substitué ou substitué une ou plusieurs fois par un halogène, alkyle $C_1$-$C_6$, alcoxy $C_1$-$C_6$ ou diméthylamino à la condition que quand chaque Hal est le chlore, Q ne doit pas être un reste pyridyle, furyle ou thiényle ni le reste 2-diméthylamino-4-pyrimidinyle.

10. Moyen selon la revendication 1 qui contient comme composant actif une 4,6-dihalo-2-($\alpha$-naphtyl)-pyrimidine, non substituée, dans laquelle Hal représente le chlore ou le brome.

11. 2-hétérocyclylpyrimidines de formule (I) selon la revendication 2 dans lesquelles Q représente un reste furyle, qui est substitué par un méthyle, méthoxy, tandis que Hal représente le chlore ou le brome.

12. 4,6-dichloro-2-(1'-naphtyl)-pyrimidine selon la revendication 2.

13. 4,6-dichloro-2-(2'-méthyl-5'-thiényl)-pyrimidine selon la revendication 2.

14. 4,6-dichloro-2-(1'-pyrrol)-pyrimidine selon la revendication 3.

15. 4,6-dichloro-2-(1-méthyl-pyrrolyl-2')-pyrimidine selon la revendication 2.

16. 4,6-dichloro-2-(5'-bromo-pyridyl-4-)-pyrimidine selon la revendication 2.

17. 4,6-dichloro-2-(2'-pyrimidinyl)-pyrimidine selon la revendication 2.

18. Moyen selon la revendication 1 qui contient la 4,6-dichloro-2-(4',6'-diméthylpyrimidinyl-2)-pyrimidine comme principe actif.

19. 2-(3,4-méthylènedioxyphényl)-4,6-dichloropyrimidine selon la revendication 3.

20. 2-(4-benzylidèneaminophényl)-4,6-dichloropyrimidine selon la revendication 3.

21. 2-[4-di(2'-hydroxyéthyl) aminophényl]-4,6-dichloropyrimidine selon la revendication 3.

22. 2-(4-phényléthinylphényl)-4,6-dichloropyrimidine selon la revendication 3.

23. 2-biphényl-4,6-dichloropyrimidine selon la revendication 3.

24. 2-(4-diphényléther)-4,6-dichloropyrimidine selon la revendication 3.

25. 2-(4-pyrrolylphényl)-4,6-dichloropyrimidine selon la revendication 3.

26. 2-(3-pyrrolylphényl)-4,6-dichloropyrimidine selon la revendication 3.

27. Procédé de préparation de 2-phényl-, 2-naphtyl- et 2-hétérocyclylpyrimidines de formule (I) selon la revendication 1, caractérisé en ce qu'on traite une 4,6-dihydroxy-2-naphtyl- ou 2-hétérocyclylpyrimidine de formule (V)

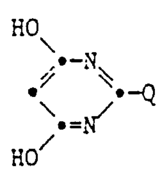

(V)

dans laquelle Q a la signification donnée dans la revendication 1, dans un solvant organique inerte avec un halogène ou un moyen cédant un halogène et qu'on isole la 4,6-dihalogéno-2-phényl-2-naphtyl- ou 2-hétérocyclyl-pyrimidine obtenue de formule (I).

28. Utilisation de 2-phényl-, 2-naphtyl- et 2-hétérocyclyl-pyrimidine de formule (I) selon la revendication 1 pour protéger des plantes cultivées contre l'action néfaste d'herbicides chloroacétanilide, acide pyridyloxyphénoxypropionique ou triazinone.

29. Utilisation de 2-phényl-, 2-naphtyl- et 2-hétérocyclylpyrimidines de formule (I) selon la revendication 1 pour protéger des céréales, des cultures de riz, maïs, sorgho et soja des dégâts phytotoxiques causés par les herbicides chloroacétanilide, acide pyridyloxyphénoxypropionique ou triazinone.

30. Procédé de protection de plantes cultivées des dommages causés par l'application d'herbicides, chloroacétanilide, acide pyridyloxyphénoxypropionique ou triazinone, caractérisé en ce qu'on traite

a) la surface cultivée pour les plantes avant ou pendant l'application de l'herbicide ou

b) la semence ou les plants des plantes ou les plantes elles-mêmes avec une quantité efficace d'une 2-phényl-, 2-naphtyl- ou 2-hétérocyclylpyrimidine de formule (I) selon la revendication 1.

31. Procédé selon les revendications 28-30 pour protéger le riz des dommages causés par les herbicides chloroacétanilide.

32. Semence de plantes de culture qui a été traitée avec une quantité efficace d'une 2-phényl-, 2-naphtyl- ou 2-hétérocyclyl-pyrimidine de formule (I) selon la revendication 1.